# FASCICULE DE BREVET EUROPEEN

(11) **EP 3 624 822 B1**
(45) Date de publication et mention de la délivrance du brevet: **23.02.2022**
(21) Numéro de dépôt: 18727702.5
(22) Date de dépôt: 14.05.2018
(51) Int. Cl.: A61K 38/06, A61K 47/64, A61K 47/26, A61P 9/10, A61K 9/00, A61K 9/08, A61K 9/20

(54) **PRINCIPE ACTIF CONSTITUE PAR UN MELANGE DE COMPOSES POLY-LYSINE ET UTILISATION DANS LA PREVENTION DES AVC ET LE TRAITEMENT DE LA PHASE INFLAMMATOIRE POST-AVC**
AKTIVE ZUSAMMENSETZUNG ENTHALTEND EINE MISCHUNG VON POLY-LYSINE VERBINDUNGEN, UND VERWENDUNG ZUR BEHANDLUNG UND VORBEUGUNG DER ENTZÜNDUNG NACH SCHLAGANFALL
ACTIVE AGENT COMPRISING A MIXTURE OF POLY-LYSINE COMPOUNDS, AND USE IN THE TREATMENT AND PREVENTION OF POST-STROKE INFLAMMATION

(30) Priorité: 16.05.2017 FR 1770496
(43) Date de publication de la demande: 25.03.2020
(73) Titulaire: PolyNeuroS, 33127 Saint Jean d'Illac (FR)
(72) Inventeur: GEFFARD, Michel, 33400 Talence (FR); MANGAS MARTIN, Arturo, 33127 Martignas-sur-Jalles (FR); VIDAL, Laetitia, 33440 Ambares et Lagrave (FR)
(74) Mandataire: Aquinov
(86) Numéro de dépôt international: PCT/EP2018/062309
(87) Numéro de publication internationale: WO 2018/210710

(56) Documents cités:
- EP-A1- 1 870 095
- WO-A2-2008/035001
- MANGAS ARTURO ET AL: "Gemst: a taylor-made combination that reverts neuroanatomical changes in stroke", EUROPEAN JOURNAL OF HISTOCHEMISTRY, vol. 61, no. 2, 3 mai 2017 (2017-05-03), pages 102-111, XP002777893, DOI: 10.4081/ejh.2017.2790
- MANGAS ARTURO ET AL: "A New Drug Candidate (GEMSP) for Multiple Sclerosis", CURRENT MEDICINAL CHEMISTRY, vol. 16, no. 25, septembre 2009 (2009-09), pages 3203-3214, XP002777894, DOI: 10.2174/092986709788803259

## Description

La présente invention concerne la prévention des risques vasculaires cérébraux chez l'être humain ou l'animal ainsi que le traitement de la phase inflammatoire qui suit un accident vasculaire cérébral.

L'accident vasculaire cérébral (AVC) est défini par l'Organisation Mondiale de la Santé comme la conséquence d'une interruption brutale de la circulation sanguine dans le cerveau. Environ 80 à 85% des AVC sont d'origine ischémique et 15 à 20% d'origine hémorragique.

Les principaux facteurs de risque à l'échelle mondiale sont la consommation de tabac, l'hypertension artérielle, l'inactivité physique, une mauvaise alimentation, l'obésité, le diabète, un taux élevé de lipides sanguins, une consommation irraisonnée d'alcool, le vieillissement, le faible niveau d'éducation sur ces sujets ainsi que des facteurs génétiques et psychologiques.

Les maladies cardiovasculaires représentent la première cause de décès dans le monde, soit près de 17 millions par an d'après un rapport de l'OMS (Mendis S., Puska P., Norrving B., World Health Organization, World Heart Federation, World Stroke Organization, 2011. Global atlas on cardiovascular disease prévention and control. World Health Organization : World Heart Fédération : World Stroke Organization, Geneva), et la part due aux AVC est prépondérante puisqu'elle constitue la seconde cause de mortalité dans la catégorie des maladies cardiovasculaires. De plus, l'AVC est la première cause de handicap acquis de l'adulte, la deuxième cause de démence et la troisième cause de mortalité en France.

Actuellement, il n'y a pas de thérapie spécifique de l'AVC. Le seul traitement efficace est l'injection intraveineuse d'un activateur tissulaire du plasminogène (t-PA) (« tissue plasminogen activator »). Cependant le t-PA a une fenêtre thérapeutique très courte et limitée de quelques heures après l'accident vasculaire cérébrale, même dans le cas ischémique. En effet, lors du processus cellulaire engagé pendant l'AVC, le t-PA n'a pas d'effet inflammatoire, œdémateux et immunitaire, et il ne peut pas être utilisé dans les cas hémorragiques.

La recherche de nouvelles solutions thérapeutiques, préventives et réparatrices est donc un sujet de santé publique majeur.

La présente invention s'inscrit dans ce contexte et son objectif est de proposer une solution thérapeutique pour prévenir et lutter contre les conséquences de l'accident vasculaire cérébral, plus efficace et applicable que les rares traitements actuels qui ne sont pas adaptés. A cet effet, l'invention a pour objet un principe actif constitué par plusieurs composés Poly-lysine, lesdits composés Poly-lysine étant constitués par au moins une petite molécule conjuguée à une Poly-lysine, ledit principe actif comprenant au moins les composés Poly-lysine suivants :
- CoenzymeQ10-Poly-lysine,
- Acide rétinoïque-Poly-lysine,
- Cystéine-Poly-lysine,
- Taurine-Poly-lysine, et
- Glutathion-Poly-lysine.

Selon l'invention, ce principe actif est efficace chez l'être humain et l'animal, dans la prévention des accidents vasculaires cérébraux et/ou dans le traitement de la phase inflammatoire qui suit un accident vasculaire cérébral.

Avantageusement, il permet en particulier :
- de maîtriser les mécanismes radicalaires, immunologiques et dégénératifs de l'AVC et,
- d'optimiser l'environnement pour l'implantation de cellules réparatrices telles que les cellules souches embryonnaires, mésenchymateuses, et totipotentes.

Le principe actif selon l'invention présente notamment un effet anti-inflammatoire sur les lésions se situant au niveau du cerveau, du dépôt de cholestérol et de la formation de caillots. Il permet de limiter la libération des radicaux libres et du glutamate afin de contrôler le système immunitaire et de protéger le cerveau, de limiter la libération des cytokines pro-inflammatoires qui vont libérer la microglie et donc de « nettoyer » les tissus artériels non irrigués.

L'invention a également pour objet une composition, pour une utilisation dans la prévention des accidents vasculaires cérébraux et/ou dans le traitement de la phase inflammatoire qui suit un AVC, comprenant au moins 7 mg en poids d'un principe actif dans la forme galénique comprimé selon l'invention.

D'autres caractéristiques et avantages ressortiront de la description en détails de l'invention qui va suivre.

### Définitions

Par « petite molécule conjuguée à une Poly-lysine », ou « petite molécule greffée sur une Poly-lysine », au sens de l'invention, on entend une molécule liée à la Poly-lysine par liaison covalente ou liaison non covalente (notamment hydrogène, ionique Van der Waals). La Poly-lysine est une macromolécule d'unités de lysines, préférentiellement de L-lysine (poly-L-lysine), linéaire ou ramifiée dont le nombre (n) de sous-unités est préférentiellement au moins de 30 lysines : n ≥ 30 lysines.

Par « prévention des AVC » au sens de l'invention, on entend neutraliser ou maîtriser les radicaux libres générés par le manque d'oxygénation du cerveau avant l'AVC en particulier en permettant à des principes actifs d'être présents dans le cerveau avant l'AVC. Plus les radicaux libres sont neutralisés dans les phases précoces de l'AVC, plus les conséquences de l'AVC sont limitées.

Par « phase d'excitotoxicité au moment de l'AVC», on entend juste après la reperfusion du tissu artériel (élimination du caillot sanguin) qui correspond à une augmentation des radicaux libres et le glutamate en tant que molécule cytotoxique déclenchant la phase d'inflammation et d'apoptose.

Par « phase inflammatoire et d'apoptose qui suit un AVC», on entend l'activation des systèmes enzymatiques tels que la voie NO synthase et la voie 2-3 indolamine dioxygénase (IDO), l'activation des cascades de réactions, telles que les caspases qui enclenchent les phases suivantes. Cette phase peut durer de quelques jours à quelques semaines en fonction de la sévérité de l'AVC.

Par « phase de réparation et de régénération qui suit un AVC» au sens de l'invention, on entend une phase d'amélioration symptomatologique du déficit neurologique causé par un AVC.

Le principe actif va donner un environnement favorable à la réparation cellulaire et au plan local, la réparation des cellules totipotentes stimulées qui vont se développer.

Par « traitement de la phase inflammatoire et d'apoptose qui suit un AVC » au sens de l'invention, on entend réduire les dommages cérébraux causés par cette phase, en diminuant le stress oxydatif et nitrosatif ainsi que la libération de cytokines inflammatoires qui permet de « nettoyer » les cellules en apoptose et en nécrose.

Par « principe actif », au sens de l'invention, on entend un principe actif thérapeutique c'est-à-dire un ensemble de molécules présentant un effet thérapeutique et en très faible proportion par rapport aux excipients.

### Description détaillée de l'invention

L'invention a donc pour objet un principe actif constitué par plusieurs composés Poly-lysine, lesdits composés Poly-lysine étant constitués par au moins une petite molécule conjuguée à une Poly-lysine, pour une utilisation chez l'être humain ou l'animal, dans la prévention des accidents vasculaires cérébraux et/ou dans le traitement de la phase inflammatoire qui suit un AVC. Le principe actif selon l'invention peut être utilisé pour protéger des effets délétères de l'AVC.

Le principe actif utile selon l'invention comprend au moins les composés Poly-lysine suivants :
- CoenzymeQ10-Poly-lysine,
- Acide rétinoïque-Poly-lysine,
- Cystéine-Poly-lysine,
- Taurine-Poly-lysine,
- Glutathion-Poly-lysine,
- α-Tocophérol-Poly-lysine,
- Acide ascorbique-Poly-lysine, et
- Méthionine-Poly-lysine.

Le principe actif selon l'invention peut être utilisé en particulier pour réduire l'inflammation au niveau des artères en prévention des accidents vasculaires cérébraux chez des sujets à risque et/ou en traitement dans la phase inflammatoire qui suit un AVC. En effet, il est capable d'agir en tant qu'inhibiteur du stress oxydatif grâce à l'apport d'antioxydants comme les vitamines.

Il peut être également utilisé pour diminuer la destruction des neurones viables par les cellules immunitaires en traitement dans la phase inflammatoire et apoptotique qui suit un AVC. En effet, il est capable d'agir sur la balance des molécules neurotoxiques/neuroprotectrices de la voie IDO (par exemple, acide 3-hydroxyanthranilique/acide kynurénique), les cellules microgliales, gliales et lymphocytaires impliquées dans le système immunitaire.

L'apport vitaminique régulier chez les patients à risque diminue la prévalence d'AVC. Plus l'AVC est pris en charge tôt avec un apport vitaminique, plus les conséquences de l'AVC sont diminuées, ainsi que la destruction du cerveau et le volume de lésions sur la zone ischémiée. Le Coenzyme Q10 présente notamment un effet neuroprotecteur grâce à son pouvoir antioxydant.

L'acide rétinoïque agit en particulier comme un agent anti-apoptotique et antioxydant.

La cystéine est notamment capable de protéger les cellules nerveuses de la toxicité du zinc libéré par les cellules nerveuses en chélatant le zinc ou en le convertissant en intermédiaire du cycle de l'acide carboxylique.

La taurine est notamment capable de protéger contre l'excitotoxicité. Elle présente un effet neuroprotecteur contre l'apoptose générée par un stress lié au réticulum endoplasmique. Elle est également capable de préserver la fonction mitochondriale et de prévenir la mort cellulaire liée à la voie mitochondriale de l'apoptose. Elle a également pour effet de maintenir l'homéostasie du contrôle neuronal et de prévenir la mort cellulaire neuronale.

Le glutathion joue un rôle important dans la défense anti-oxydante, le métabolisme nutritif et dans la régulation des évènement cellulaires.

Ces différents constituants du principe actif selon l'invention agissent ensemble et de façon synergique pour permettre un effet de prévention de l'AVC et de traitement dans la phase inflammatoire qui suit un AVC. En effet, le principe actif selon l'invention est capable de générer les conditions environnementales nécessaires pour la réparation, la diminution des molécules neurotoxiques, et de diminuer l'inflammation. Cela permet un meilleur contrôle de la zone périphérique ischémiée dans laquelle la mort neuronale apoptotique s'est produite. L'association des composés poly-lysines spécifiques constituant le principe actif (polycomplexe) selon l'invention permet de réparer les dommages cérébraux par la diminution du stress oxydatif et nitrosatif, de diminuer la libération de cytokines inflammatoires qui permet de « nettoyer » les cellules en apoptose et en nécrose. Le principe actif selon l'invention régule ainsi l'activation immunitaire, diminue les effets délétères par la diminution des marqueurs IBA1 (macrophage), CD45 (pan leucocyte), CD11b (microglie activée), GFAP (astrogliose). Les molécules de la voie IDO telles que les acides 3-hydroxyanthranilique et kynurénique sont surexprimées en cas d'AVC (voir résultats sur modèle animal utilisé dans la partie expérimentale, qui consiste à interrompre les flux sanguins pendant environ 1 heure et suivi d'une re-perfusion du tissu cérébral) et l'utilisation du principe actif selon l'invention permet d'agir dans la modulation de cette voie métabolique. Par ailleurs, les composés poly-lysine constituant le principe actif selon l'invention, traversent directement la paroi vasculaire sublinguale et ainsi sont distribués par le sang au niveau des lésions dans l'objectif de neutraliser les espèces radicalaires et éviter ainsi les modifications protéiques endogènes, et donc la mort neuronale.

En outre, le fait que ces différentes molécules soient conjuguées à la Poly-Lysine permet de les rendre actives. En effet, la Poly-lysine est un polypeptide cationique alpha- Poly-lysine, dont les caractéristiques de linéarité, de non immunogénicité, de structure secondaire en « random coil », lui confèrent la propriété de rendre active une petite molécule une fois liée à la Poly-lysine.

L'effet de prévention du principe actif selon l'invention peut être avant un premier AVC chez une personne à risque, ou après un AVC en prévention d'un autre AVC.

En plus de ces composés Poly-lysine :
- CoenzymeQ10-Poly-lysine,
- Acide rétinoïque-Poly-lysine,
- Cystéine-Poly-lysine,
- Taurine-Poly-lysine,
- Glutathion-Poly-lysine,
- α-Tocophérol-Poly-lysine,
- Acide ascorbique-Poly-lysine, et
- Méthionine-Poly-lysine.

Le principe actif selon l'invention peut comprendre d'autres composés Poly-lysine. L'a-Tocophérol présente un effet antioxydant important. Cet effet est augmenté en présence d'acide ascorbique.

De plus, l'acide ascorbique en tant que tel est notamment capable de protéger les neurones de l'excitotoxicité et de prévenir les dommages cellulaires induits par le glutamate et la mort cellulaire.

La méthionine agit comme un système de défense endogène antioxydant dans les cellules et est également un précurseur du glutathion.

En combinaison avec les autres composés spécifiques du principe actif selon l'invention, ils agissent en synergie pour un effet sur la prévention de l'AVC et le traitement dans la phase inflammatoire qui suit un AVC.

Selon un mode de réalisation, et de façon préférée, le principe actif selon l'invention est constitué exclusivement par les composés Poly-lysine suivants :
- CoenzymeQ10-Poly-lysine
- Acide rétinoïque-Poly-lysine
- Cystéine-Poly-lysine
- Taurine-Poly-lysine
- Glutathion-Poly-lysine
- α-Tocophérol-Poly-lysine
- Acide ascorbique-Poly-lysine
- Méthionine-Poly-lysine.

Dans le principe actif selon l'invention, quelle que soit sa composition, la Poly-lysine constituant les composés Poly-lysine est préférentiellement la Poly-L-Lysine. Les systèmes ayant une activité biologique sont très généralement constitués d'acides aminés en configuration L, dits « naturels ».

Dans le principe actif, chacune des petites molécules constituant les composés Poly-lysine, est présente à des concentrations préférentiellement comprises entre 1×10⁻⁴ et 1×10⁻³ M. Selon un mode de réalisation adapté, tous les composés Poly-lysine sont à la même concentration en petites molécules, ou à une concentration quasi-identique pour obtenir le meilleur effet thérapeutique.

Le principe actif selon l'invention peut-être obtenu par un procédé comprenant les étapes suivantes :
- Synthèse des composés Poly-lysine
- Mélange des composés Poly-lysine.

La synthèse des composés Poly-lysine peut être réalisée par greffage des petites molécules sur des Poly-lysine, selon les connaissances de l'homme du métier.

Le composé Acide rétinoïque-Poly-lysine peut par exemple être obtenu selon le procédé décrit ci-après :
- l'acide rétinoïque (rétinoyl) est activé par le chloroformiate d'éthyl (ECF) qui réagit comme agent couplant par activation de l'acide carboxylique de la vitamine,
- l'activation est facilitée par l'ajout d'un solvant polaire, le produit intermédiaire (rétinoyl-ECF) réagit sur le groupement epsilon aminé du résidu lysyl de la Poly-lysine, par la formation d'une liaison amide,
- puis le composé rétinoyl- Poly-lysine est purifié idéalement par ultrafiltration tangentielle, puis lyophilisé si nécessaire,
- l'acide rétinoïque est alors greffé par liaison covalente à la Poly-Lysine.

Les composés Coenzyme Q10-Poly-lysine et α-tocophérol-Poly-lysine peuvent être fabriqués de la même façon.

Le composé Acide ascorbique-Poly-lysine peut par exemple être obtenu selon le procédé décrit ci-après :
- l'acide ascorbique (ascorbyl) est activé par le carbodiimide (EDC) qui réagit comme agent couplant par activation de l'acide carboxylique de la vitamine,
- cette activation est facilitée par l'ajout d'un catalyseur, le produit intermédiaire (ascorbyl-EDC) réagit sur le groupement epsilon aminé du résidu lysyl de la Poly-lysine, par la formation d'une liaison amide,
- puis le composé ascorbyl-EDC-Poly-lysine est purifié idéalement par ultrafiltration tangentielle, puis lyophilisé si nécessaire,
- l'acide ascorbique est alors greffé par liaison covalente à la Poly-lysine.

Le composé Cystéine-Poly-lysine peut par exemple être obtenu selon le procédé décrit ci-après :
- la cystéine est activée par le glutaraldéhyde (G) qui réagit comme agent couplant par activation de l'amine de l'acide aminé,
- le produit intermédiaire (cystéine-G) réagit sur le groupement epsilon aminé du résidu lysyl par la formation d'une liaison amide,
- puis le composé cystéine-G-Poly-lysine est purifié idéalement par ultrafiltration tangentielle, puis lyophilisé si nécessaire,
- la cystéine est alors greffée par liaison covalente à la Poly-lysine.

Les composés Méthionine-Poly-lysine, Taurine-Poly-lysine et Glutathion-Poly-lysine peuvent être fabriqués de la même façon.

Les composés Poly-lysine sont ensuite mélangés pour fabriquer le principe actif selon l'invention. Préférentiellement, le mélange est réalisé par la mise en œuvre d'un procédé comprenant les étapes suivantes :
- récupération des quantités indiquées de chaque composé Poly-lysine sous forme liquide,
- calcul pour chaque composé Poly-lysine de la concentration en petites molécules, à partir d'un volume donné à prélever par rapport à la concentration finale et au volume final souhaités du mélange,
- avant prélèvement, les composés Poly-lysine sont agités manuellement ou mécaniquement,
- les composés Poly-lysine en phase liquide sont ensuite mélangés après pipetage sous agitation magnétique,
- l'émulsion du mélange liquide est réalisée à des températures comprises entre 18 et 22°C, par agitation mécanique vigoureuse entre 60 et 400 tours/min,
- le mélange obtenu est congelé puis lyophilisé pour obtenir une poudre ou bien le mélange obtenu est utilisé sous forme liquide.

Le principe actif selon l'invention peut en effet se présenter sous forme sèche (préférentiellement lyophilisée) ou sous forme liquide (solution).

Avantageusement, le principe actif selon l'invention est très efficace tout en ne présentant aucun effet secondaire indésirable. En effet, des expérimentations animales ont validé l'absence de toxicité et d'effets secondaires indésirables.

Pour son utilisation dans la prévention des AVC, le principe actif selon l'invention est préférentiellement utilisé entre 5 et 15 mg par jour, en particulier entre 7 et 10 mg, sous forme sèche.

Les personnes visées pour la prévention et la prise du principe actif selon l'invention dans ce cadre sont des personnes présentant un risque d'AVC, à savoir en particulier des personnes en hypercholestérolémie, en hypertension, des personnes à risque ayant déjà eu un AVC, des personnes sédentaires cumulant avec la prise d'alcool, de tabac et autres facteurs.

Pour son utilisation dans le traitement de la phase inflammatoire qui suit un AVC, le principe actif selon l'invention est utilisé entre 5 et 15 mg par jour, soit préférentiellement entre 7 et 10 mg., sous forme sèche ou sous forme injectable suivant la sévérité de l'AVC.

Les personnes visées pour ce traitement et la prise du principe actif selon l'invention dans ce cadre sont des personnes ayant eu un AVC pendant une période allant préférentiellement immédiatement après l'AVC jusqu'à 2 semaines et de façon continue pour une période indéterminée, période où le pronostic vital est en jeu.

Les principes actifs et compositions selon l'invention sont particulièrement efficaces dans le cadre du traitement en phase tardive de l'AVC.

Le principe actif selon l'invention peut être associé à d'autres thérapies, telles que par exemple le plasminogène et/ou d'autres thérapies nouvelles, le principe actif selon l'invention étant compatible avec d'autres principes actifs et non immunogénique.

Avantageusement, il peut être utilisé sur de longues périodes de temps, ce qui présente un avantage car il n'y a pas d'effets secondaires indésirables, ce qui est important dans le cas de l'AVC, car sa prévalence augmente avec l'âge et avec un premier épisode d'AVC.

Le principe actif est très préférentiellement utilisé dans une composition.

L'invention a par conséquent également pour objet une composition pour une utilisation chez l'être humain ou l'animal dans la prévention des accidents vasculaires cérébraux et/ou dans le traitement de la phase inflammatoire qui suit un AVC, comprenant au moins 7 mg en poids d'un principe actif dans la forme galénique comprimé selon l'invention.

La composition peut être sous forme solide, en particulier sous forme sèche. Il peut s'agir par exemple d'un comprimé à avaler, de comprimé sublingual, de gélule gastro-résistante, de poudre, de patch dermique ou de suppositoire. La composition sous forme sèche comprend au moins un excipient, comme par exemple un support d'atomisation tel que la maltodextrine par exemple, ou la cellulose microcristalline ou le stéarate de magnésium ou le polyéthylène glycol.

Lorsque la composition est sous forme sèche, le principe actif selon l'invention est préférentiellement présent entre 5 et 15 mg pour 100 mg de composition, encore plus préférentiellement entre 7 et 10 mg pour 100 mg de composition.

La composition peut être sous forme liquide. En particulier, elle peut se présenter sous forme d'une solution buvable ou solution en suspension ou injectable. Il peut s'agir comme excipient majoritaire par exemple le chlorure de sodium et de l'eau purifiée, ou le sorbitol et de l'eau purifiée ou la saccharine et de l'eau purifiée.

Lorsque la composition est sous forme liquide, le principe actif selon l'invention est préférentiellement présent entre 5 et 15 mg par ml de composition, encore plus préférentiellement entre 7 et 10 mg par ml de composition.

L'invention est à présent illustrée par des exemples et des résultats d'essais démontrant l'efficacité du principe actif.

### Exemples de principes actifs (polycomplexes) selon l'invention

### Procédé de réalisation des polycomplexes

Les polycomplexes sont obtenus selon le procédé suivant :
- récupération des quantités indiquées de chaque composé Poly-lysine sous forme liquide ;
- obtention pour chaque composé Poly-lysine avec son calcul de concentration en petites molécules, d'un volume donné à prélever par rapport à la concentration finale et au volume final souhaités en polycomplexe, à savoir de 1000 à 10 000 mL;
- avant prélèvement, les composés Poly-lysine sont agités manuellement ou mécaniquement ;
- les composés Poly-lysine en phase liquide sont ensuite mélangés après pipetage sous agitation magnétique ;
- l'émulsion du mélange liquide est réalisée à des températures comprises entre 18 et 22°C, par agitation mécanique vigoureuse à 100 tours/min ;
- l'émulsion du mélange est homogénéisée aussi par sonification ;
- le mélange obtenu est congelé puis lyophilisé pour obtenir une poudre.

### Exemple 1 : exemple de polycomplexe 1 (principe actif)

| Composés Poly-L-Lysine | Concentration finale (en mol/L) En petites molécules greffées |
|---|---|
| COENZYMEQ10- Poly-L-Lysine | 2 ×10⁻⁴ |
| ACIDE RETINOÏQUE- Poly-L-Lysine | 2 ×10⁻⁴ |
| CYSTEINE- Poly-L-Lysine | 2 ×10⁻⁴ |
| TAURINE- Poly-L-Lysine | 2 ×10⁻⁴ |
| GLUTATHION- Poly-L-Lysine | 2 ×10⁻⁴ |

| | |
|---|---|
| Poids : 4,7 mg par comprimé | |

### Exemple 2 : exemple de polycomplexe 2 (principe actif) selon l'invention

| Composés Poly-L-Lysine | Concentration finale (en mol/L) En petites molécules greffées |
|---|---|
| COENZYMEQ10- Poly-L-Lysine | 2 ×10⁻⁴ |
| ACIDE RETINOÏQUE- Poly-L-Lysine | 2 ×10⁻⁴ |
| CYSTEINE- Poly-L-Lysine | 2 ×10⁻⁴ |
| TAURINE- Poly-L-Lysine | 2 ×10⁻⁴ |
| GLUTATHION- Poly-L-Lysine | 2 ×10⁻⁴ |
| TOCOPHEROL- Poly-L-Lysine | 2 ×10⁻⁴ |
| ACIDE ASCORBIQUE- Poly-L-Lysine | 2 ×10⁻⁴ |
| METHIONINE- Poly-L-Lysine | 2 ×10⁻⁴ |

| | |
|---|---|
| Poids : 7,5 mg par comprimé | |

### Exemple 3: exemple de polycomplexe 3 (principe actif) de l'art antérieur

| Composés Poly-L-Lysine | Concentration finale (en mol/L) En petites molécules greffées |
|---|---|
| COENZYMEQ10- Poly-L-Lysine | 4 ×10⁻⁴ |
| ACIDE RETINOÏQUE- Poly-L-Lysine | 4 ×10⁻⁴ |
| TOCOPHEROL- Poly-L-Lysine | 4 ×10⁻⁴ |
| ACIDE ASCORBIQUE- Poly-L-Lysine | 4 ×10⁻⁴ |

| | |
|---|---|
| Poids : 7,5 mg par comprimé | |

### Exemple 4 : exemple de polycomplexe 4 (principe actif) de l'art antérieur

| Composés Poly-L-Lysine | Concentration finale (en mol/L) En petites molécules greffées |
|---|---|
| CYSTEINE- Poly-L-Lysine | 4 ×10⁻⁴ |
| TAURINE- Poly-L-Lysine | 4 ×10⁻⁴ |
| GLUTATHION- Poly-L-Lysine | 4 ×10⁻⁴ |
| METHIONINE- Poly-L-Lysine | 4 ×10⁻⁴ |

| | |
|---|---|
| Poids : 7,5 mg par comprimé | |

### Exemple 5 : exemple de composition comprenant le polycomplexe 2 avec des excipients sous forme solide.

La composition de l'exemple 5 pour une unité thérapeutique (1 comprimé) est la suivante :

| Composants | **Teneur** (%) |
|---|---|
| Mannitol (pour compression directe) | 56,5 |
| Amidon prégélatinisé | 14 |
| Cellulose microcristalline | 10 |
| PEG | 5 |
| PVP K30 | 2 |
| Talc | 2 |
| Stéarate de Magnésium | 1 |
| Silice amorphe | 1 |
| Lévilite | 1 |
| **Total excipients** | **92,5** |
| **Principe actif selon l'invention** : **exemple 2** | **7,5** |
| **Total** | **100** |

La composition selon l'invention est présente sous forme d'un comprimé sublingual de 100 mg.

La poudre contenant les principes actifs et les excipients est mise sous presse pour constituer le comprimé sublingual de sorte qu'il puisse se dissoudre en 3 à 10 minutes.

La posologie pour ce comprimé prescrit seul est de 2 comprimés 3 fois par jour, deux jours sur trois, soit 90 mg par mois d'antioxydants et pièges à radicaux libres.

### Exemple 6 : exemple de composition comprenant le polycomplexe 2 avec des excipients en solution buvable

La composition de l'exemple 6 pour une unité thérapeutique (100 mL) est la suivante :

| **Composants** | **Teneur** (%) |
|---|---|
| Eau Purifiée | 74 |
| Saccharine | 5 |
| Sorbitol | 4 |
| Arôme | 2 |
| **Total excipients** | **85** |
| **Principe actif selon l'invention** : **exemple 2** | **15** |
| **Total** | **100** |

### Essais démontrant l'efficacité de l'invention

Des essais ont été réalisés sur un modèle d'AVC chez le rat qui consiste à interrompre le flux sanguin pendant environ une heure suivi d'une re-perfusion du tissu cérébral (Mangas A, Yajeya J, González N, Ruiz I, Geffard M, Coveñas R. 3-hydroxi-anthranilic acid is early expressed in stroke Eur J Histochem 2016; 60: 2709 ; Mangas A, Yajeya J, González N, Ruiz I, Duleu S, Geffard M, Coveñas R. Overexpression of kynurenic acid in stroke: an endogenous neuroprotector? Ann Anat 2017 ; 211:33-8. Ulu 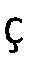 K, Miranpuri A, Kujoth GC, Aktüre E, Ba 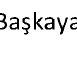 kaya MK. Focal cerebral ischemia model by endovascular suture occlusion of the middle cerebral artery in the rat J Vis Exp. 2011 Feb 5;(48). pii: 1978)

Le protocole opératoire est défini en suivant.

On introduit un filament en nylon avec un embout en silicone au niveau de l'artère carotide interne jusqu'à l'artère médiane en interrompant le flux sanguin pendant une heure. Après une heure, le filament est retiré et la circulation sanguine est rétablie. Pendant la phase de réanimation, le principe actif selon l'invention est injecté.

L'objectif des essais est de démontrer que les animaux traités après la re-perfusion pendant 21 jours présentent une réversion des effets délétères causés par la privation du flux sanguin.

Les résultats obtenus sont présentés dans le tableau ci-après :

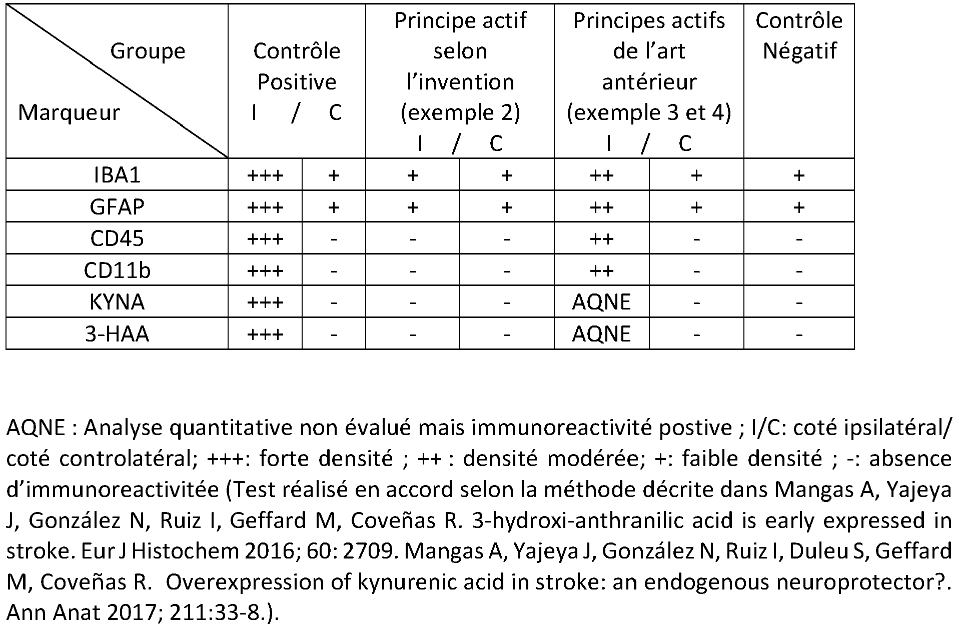

On constate que les animaux non traités avec l'exemple 2 (invention) présentent une région endommagée (striatum et ou cortex) qui est caractérisée par une surexpression des marqueurs IBA1 et GFAP dans la région de la lésion en comparaison avec l'hémisphère controlatéral. Ce n'est pas le cas avec les animaux du groupe contrôle négatif du modèle ainsi que les animaux traités avec l'invention.

La présence de microglie activée (évaluée avec le marqueur CD11b) et l'infiltration leucocytaire (évaluée avec le marqueur pan-leucocytique CD45) est notable seulement dans la région de la lésion des animaux traités avec le véhicule à 21 jours et ceux traités avec les exemples 3 et 4, les animaux sains (contrôle négatif) et les animaux traités avec le principe actif (exemple 2) ne présentent pas cette infiltration. Il faut remarquer que dans la même région endommagée, il y a une co-existence des métabolites de la voie IDO (acides 3-hydroxyanthranilique et kynurénique) dans les astrocytes. Il est connu que l'acide 3-hydroxyanthranilique a des effets neurotoxiques et l'acide kynurénique a des effets neuroprotecteurs. Donc la sur-expression de ces 2 molécules est due à l'activation de la voie IDO dans le présent modèle d'AVC (Mangas et al., 2016; 2017). Cette sur-expression est inhibée après traitement avec le principe actif (exemple 2) selon l'invention. Ainsi, le principe actif selon l'invention restaure les conditions pathologiques induites par le modèle d'AVC aux conditions dites « normales ». De plus, il permet d'abolir l'infiltration dans le cerveau, l'activation de la microglie, l'astrogliose et de moduler la voie IDO, au moins pour l'acide 3-hydroxyanthranilique et l'acide kynurénique.

Les composés Poly-lysine constituant le principe actif selon l'invention (exemple 2) arrivent ainsi à neutraliser les effets délétères 21 jours après l'AVC, en comparaison avec les animaux non-traités (contrôle positif) et les animaux traités avec l'art antérieur (exemples 3 et 4).

## Revendications

1. Principe actif constitué par plusieurs composés Poly-lysine, lesdits composés Poly-lysine étant constitués par au moins une petite molécule conjuguée à une Poly-lysine, ledit principe actif comprenant au moins les composés Poly-lysine suivants :
- CoenzymeQ10-Poly-lysine,
- Acide rétinoïque-Poly-lysine,
- Cystéine-Poly-lysine,
- Taurine-Poly-lysine, et
- Glutathion-Poly-lysine,
- α-Tocophérol-Poly-lysine
- Acide ascorbique-Poly-lysine
- Méthionine-Poly-lysine.
pour une utilisation chez l'être humain ou l'animal, dans la prévention des accidents vasculaires cérébraux et/ou dans le traitement de la phase inflammatoire qui suit un accident vasculaire cérébral.

2. Principe actif pour une utilisation selon la revendication 1, pour réduire l'inflammation au niveau des artères en prévention des accidents vasculaires cérébraux chez des sujets à risque et/ou en traitement dans la phase inflammatoire qui suit un accident vasculaire cérébral.

3. Principe actif pour une utilisation selon la revendication 1 ou 2, pour diminuer la destruction des neurones viables par les cellules immunitaires en traitement dans la phase inflammatoire et apoptotique qui suit un accident vasculaire cérébral.

4. Principe actif pour une utilisation selon l'une des précédentes revendications, **caractérisé en ce qu'**il est constitué exclusivement par les composés Poly-lysine suivants :
- CoenzymeQ10-Poly-lysine
- Acide rétinoïque-Poly-lysine
- Cystéine-Poly-lysine
- Taurine-Poly-lysine
- Glutathion-Poly-lysine
- α-Tocophérol-Poly-lysine
- Acide ascorbique-Poly-lysine
- Méthionine-Poly-lysine.

5. Principe actif pour une utilisation selon l'une des précédentes revendications, **caractérisé en ce que** la Poly-lysine est la Poly-L-lysine.

6. Principe actif pour une utilisation selon l'une des précédentes revendications, **caractérisé en ce que** les concentrations en chacune desdites petites molécules sont comprises entre 1×10⁻⁴ et 1×10⁻³ M.

7. Principe actif pour une utilisation selon l'une des précédentes revendications, **caractérisé en ce que** tous les composés Poly-lysine sont à la même concentration en petites molécules.

8. Composition pour une utilisation chez l'être humain ou l'animal dans la prévention des accidents vasculaires cérébraux et/ou dans le traitement de la phase inflammatoire qui suit un accident vasculaire cérébral, comprenant au moins 7 mg en poids d'un principe actif selon l'une des précédentes revendications.

9. Composition pour une utilisation selon la précédente revendication, **caractérisée en ce qu'**elle se présente sous forme sèche.

10. Composition pour une utilisation selon la précédente revendication, **caractérisée en ce qu'**elle se présente sous forme de comprimé à avaler, de comprimé sublingual, de gélule gastro-résistante, de poudre, de patch dermique ou de suppositoire.

11. Composition pour une utilisation selon l'une des revendications 9 ou 10, **caractérisée en ce que** le principe actif est présent entre 5 et 15 mg pour 100 mg de composition.

12. Composition pour une utilisation selon la revendication 8, **caractérisée en ce qu'**elle se présente sous forme liquide.

13. Composition pour une utilisation selon la précédente revendication, **caractérisée en ce qu'**elle se présente sous forme d'une solution buvable ou injectable.

14. Composition pour une utilisation selon l'une des revendications 12 ou 13, **caractérisée en ce que** le principe actif thérapeutique est présent à une concentration comprise entre 5 et 15mg par ml de composition.

## Patentansprüche

1. Wirkstoff, bestehend aus mehreren Polylysin-Verbindungen, wobei die Polylysin-Verbindungen aus mindestens einem kleinen Molekül bestehen, das mit einem Polylysin konjugiert ist, wobei der Wirkstoff mindestens die folgenden Polylysin-Verbindungen umfasst:
- CoenzymQ10-Polylysin,
- Retinsäure-Polylysin,
- Cystein-Polylysin,
- Taurin-Polylysin, und
- Glutathion-Polylysin,
- α-Tocopherol-Polylysin
- Ascorbinsäure-Polylysin
- Methionin-Polylysin.
zur Verwendung bei Menschen oder Tieren zur Schlaganfallprävention und/oder zur Behandlung der entzündlichen Phase nach einem Schlaganfall.

2. Wirkstoff zur Verwendung nach Anspruch 1, um die Entzündung in den Arterien als Schlaganfallprävention bei Risikopersonen und/oder als Behandlung in der entzündlichen Phase nach einem Schlaganfall zu reduzieren.

3. Wirkstoff zur Verwendung nach Anspruch 1 oder 2, um die Zerstörung lebensfähiger Neuronen durch Immunzellen bei der Behandlung in der entzündlichen und apoptotischen Phase nach einem Schlaganfall zu verringern.

4. Wirkstoff zur Verwendung nach einem der vorhergehenden Ansprüche, **dadurch gekennzeichnet, dass** er ausschließlich aus den folgenden Polylysin-Verbindungen besteht:
- CoenzymQ10-Polylysin
- Retinsäure-Polylysin
- Cystein-Polylysin
- Taurin-Polylysin
- Glutathion-Polylysin
- α-Tocopherol-Polylysin
- Ascorbinsäure-Polylysin
- Methionin-Polylysin.

5. Wirkstoff zur Verwendung nach einem der vorhergehenden Ansprüche, **dadurch gekennzeichnet, dass** das Polylysin Poly-L-Lysin ist.

6. Wirkstoff zur Verwendung nach einem der vorhergehenden Ansprüche, **dadurch gekennzeichnet, dass** die Konzentrationen jedes der kleinen Moleküle im Bereich von 1×10⁻⁴ bis 1×10⁻³ M liegen.

7. Wirkstoff zur Verwendung nach einem der vorhergehenden Ansprüche, **dadurch gekennzeichnet, dass** alle Polylysin-Verbindungen die gleiche Konzentration an kleinen Molekülen aufweisen.

8. Zusammensetzung zur Verwendung bei Menschen oder Tieren zur Schlaganfallprävention und/oder zur Behandlung der entzündlichen Phase nach einem Schlaganfall, umfassend mindestens 7 mg, bezogen auf das Gewicht, eines Wirkstoffs nach einem der vorhergehenden Ansprüche.

9. Zusammensetzung zur Verwendung nach dem vorhergehenden Anspruch, **dadurch gekennzeichnet, dass** sie in trockener Form vorliegt.

10. Zusammensetzung zur Verwendung nach dem vorhergehenden Anspruch, **dadurch gekennzeichnet, dass** sie in Form einer Schlucktablette, einer Sublingualtablette, einer magensaftresistenten Kapsel, eines Pulvers, eines Hautpflasters oder eines Suppositoriums vorliegt.

11. Zusammensetzung zur Verwendung nach einem der Ansprüche 9 oder 10, **dadurch gekennzeichnet, dass** der Wirkstoff zwischen 5 und 15 mg pro 100 mg der Zusammensetzung vorhanden ist.

12. Zusammensetzung zur Verwendung nach Anspruch 8, **dadurch gekennzeichnet, dass** sie in flüssiger Form vorliegt.

13. Zusammensetzung zur Verwendung nach dem vorhergehenden Anspruch, **dadurch gekennzeichnet, dass** sie in Form einer trinkbaren oder injizierbaren Lösung vorliegt.

14. Zusammensetzung zur Verwendung nach einem der Ansprüche 12 oder 13, **dadurch gekennzeichnet, dass** der therapeutische Wirkstoff in einer Konzentration zwischen 5 und 15 mg pro ml der Zusammensetzung vorliegt.

## Claims

1. Active ingredient consisting of a plurality of polylysine compounds, said polylysine compounds consisting of at least one small molecule conjugated to a polylysine, said active ingredient comprising at least the following polylysine compounds:
- coenzyme Q10-polylysine,
- retinoic acid-polylysine,
- cysteine-polylysine,
- taurine-polylysine, and
- glutathione-polylysine,
- α-tocopherol-polylysine,
- ascorbic acid-polylysine,
- methionine-polylysine,
for use in humans or animals, to prevent strokes and/or treat the inflammatory phase following a stroke.

2. Active ingredient for use according to claim 1, for reducing inflammation in the arteries to prevent strokes in patients at risk and/or treat the inflammatory phase following a stroke.

3. Active ingredient for use according to claim 1 or 2, for reducing the destruction of viable neurons by the immune cells during treatment in the inflammatory and apoptotic phase following a stroke.

4. Active ingredient for use according to one of the preceding claims, **characterized in that** it consists exclusively of the following polylysine compounds:
- coenzyme Q10-polylysine,
- retinoic acid-polylysine,
- cysteine-polylysine,
- taurine-polylysine,
- glutathione-polylysine,
- α-tocopherol-polylysine,
- ascorbic acid-polylysine,
- methionine-polylysine.

5. Active ingredient for use according to one of the preceding claims, **characterized in that** the polylysine is poly-L-lysine.

6. Active ingredient for use according to one of the preceding claims, **characterized in that** the concentrations of each of said small molecules are between 1×10⁻⁴ and 1×10⁻³ M.

7. Active ingredient for use according to one of the preceding claims, **characterized in that** all the polylysine compounds have the same concentration of small molecules.

8. Composition for use in humans or animals, to prevent strokes and/or treat the inflammatory phase following a stroke, comprising at least 7 mg by weight of an active ingredient according to one of the preceding claims.

9. Composition for use according to the preceding claim, **characterized in that** it is in dry form.

10. Composition for use according to the preceding claim, **characterized in that** it is in the form of a tablet to be swallowed, a sublingual tablet, a gastro-resistant capsule, a powder, a dermal patch, or a suppository.

11. Composition for use according to one of claims 9 or 10, **characterized in that** the active ingredient is present at between 5 and 15 mg per 100 mg of composition.

12. Composition for use according to claim 8, **characterized in that** it is in liquid form.

13. Composition for use according to the preceding claim, **characterized in that** it is in the form of an oral or injectable solution.

14. Composition for use according to one of claims 12 or 13, **characterized in that** the therapeutic active ingredient is present at a concentration of between 5 and 15 mg per ml of composition.
